# EUROPEAN PATENT SPECIFICATION

(11) **EP 1 058 523 B1**
(45) Date of publication and mention of the grant of the patent: **10.08.2005**
(21) Application number: 99913214.5
(22) Date of filing: 03.03.1999
(51) Int. Cl.: A61F 2/40, A61F 2/46

(54) **AN IMPROVED MODULAR JOINT PROSTHESIS**
MODULARE GELENKPROTHESE
PROTHESE MODULAIRE AMELIOREE DE L'ARTICULATION

(30) Priority: 03.03.1998 EP 98870043
(43) Date of publication of application: 13.12.2000
(73) Proprietor: De Wilde, Lieven, 9000 Gent (BE)
(72) Inventor: De Wilde, Lieven, 9000 Gent (BE)
(74) Representative: Thivillier, Patrick
(86) International application number: PCT/EP1999/001356
(87) International publication number: WO 1999/044546

(56) References cited:
- EP-A- 0 329 854
- EP-A- 0 547 369
- DE-A- 2 702 773
- DE-A- 19 548 154
- DE-U- 9 312 218
- US-A- 3 979 778
- US-A- 5 064 427

## Description

The present invention relates to a prosthesis for a shoulder joint.

A prosthesis as defined in the preamble of claim 1 is disclosed in DE-A-195 48 154.

### TECHNICAL BACKGROUND

Of all the fractures of the upper end of the human or animal humerus, the most serious are so-called four-part fractures in accordance with the classification by Neer (see the article by C. S. Neer, "Displaced Proximal Humeral Fractures", in the Journal of Bone and Joint Surgery, vol. 52-A, No. 6, Sept. 1970, pp 1077 to 1089), particularly when these fractures are displaced. It is generally accepted that only displaced fractures require surgical treatment, i.e. when the bone fragments are separated by more than 10 mm or angulated by more than 45 degrees.

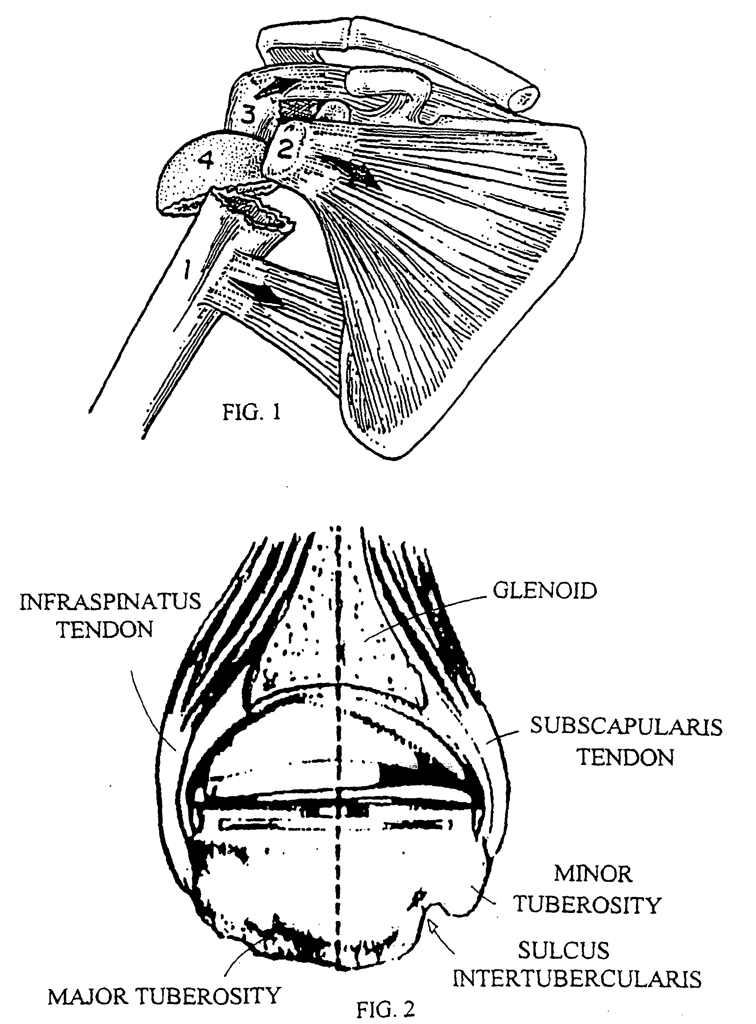

Fig. 1 illustrates the role played by the muscles in such a fracture. Each group of muscles pulls the bone fragments in a different direction. The diaphysis 1 is drawn inwards by the pectoralis major and latissimus dorsi muscles and is separated from the epiphysis at the anatomical neck or surgical neck. The lesser tuberosity 2 is retracted inwards and forwards by the subscapularis muscle. The greater tuberosity 3 is retracted inwards, upwards and backwards by the supraspinatus, infraspinatus and teres minor muscles. The tuberosities 2 and 3 may remain intact or be fragmented and may be retracted or remain in continuity with one another (impacted form). The humeral joint surface 4 is no longer in contact with the glenoid. It is separated from the two tuberosities 2 and 3 and may undergo varus or valgus displacement, dislocate forwards or backwards, upwards or downwards, usually resulting in devasculation. It may be present as an impaction or a separate fracture.

The results of conservative treatment and surgical reduction of such injuries have been discouraging because of pseudoarthrosis, malunions and above all because of the high incidence of avascular necrosis of the humeral head. Although an attempt can be made sometimes to conserve the humeral head, the preferred treatment has been the replacement of the humeral head by a non-constrained prosthesis with osteosysnthesis of the tuberosities as proposed by C. S. Neer in 1951. However, the mobility of the shoulder is often disappointing after using a prosthesis for a displaced fracture of the upper end of the humerus. Even when the result is satisfactory with respect to pain, active elevation often remains limited. The reconstruction of the upper end of the humerus using a non-constrained prosthesis poses several problems: the correlation between the quality of the anatomical and clinical results indicates that certain insufficient functional results following hemiarthroplastie for four-part fractures are due to insufficient restoration of the normal anatomy of the proximal humerus. Moreover, too little or no attention was paid to the relationship between the proximal humeral anatomy and the glenoid anatomy.

Any significant change in the normal anatomy of the proximal humerus and/or the glenoid causes a modification in the balance of the forces generated by contraction of the muscles - the so-called rotator cuff force couples, and a modification in the lever arms of the deltoid muscle in the sagittal, scapular and coronal plane. The most common technical surgical errors involve restoration of the correct length and medial offset of the humerus, the adjustment of retroversion, and the reconstruction of the tuberosities resulting in the restoration of the balance of the rotator cuff force couples.

There are many designs of modular shoulder joint prostheses and theoretically the proximal humeral anatomy can be restored perfectly in all cases. However, although this may be true for reconstructive surgery of the glenohumeral joint for degenerative or inflammatory arthritis, until now there is no guarantee of a perfect restoration of the caput humeri and the surrounding rotor cuff tendon with respect to the glenoid anatomy in fracture treatment of the proximal humerus because the landmarks necessary for the restoration have been lost as a result of the fracture.

One reason for these problems may lie in the well established adoption in informed medical circles of surgical techniques for the reconstruction of the proximal humerus which are derived from those used successfully for the reconstruction of the proximal femur of the hip. Accordingly, in proximal humeral fracture the restoration of the caput humeri currently builds from the fractured proximal humerus towards the glenoid in the same way as solid osteosynthesis of the caput femoris and the proximal femur in hip fracture is based on the femoral shaft. Hence, a conventional shoulder prosthesis still has a similar design to that of a femoral stem prosthesis. However, there are significant biomedical differences between the shoulder and the hip joint.

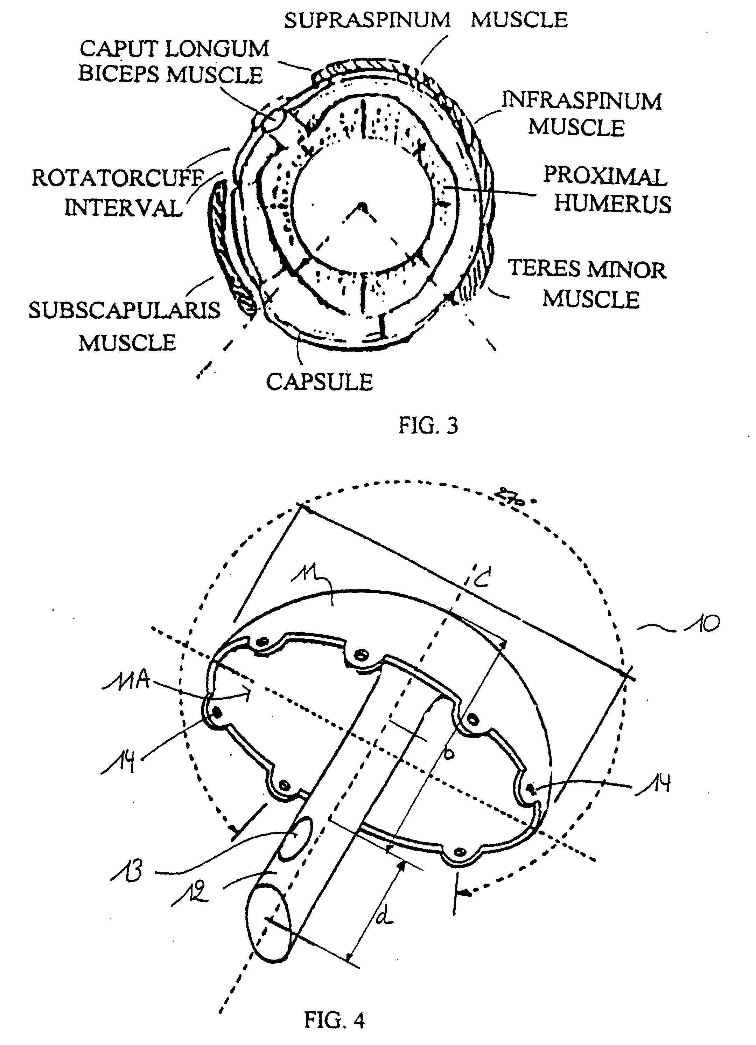

The consequences of failing to find an optimal solution are as follows.
1) When the rotator force couple is insufficiently restored a biomedical failure of the shoulder joint activity will occur. The rotator force couple generated by the muscles on either side of the head centres the head of the humerus in an active way with respect to the glenoid as shown diagramatically in Fig. 2 which is a cross-section through the gleno-humeral joint at the centre of rotation. The tendons of the teres minor, infraspinatus and supraspinatus muscles are firmly attached to the greater tuberosity and the tendon of the subscapularis muscle is firmly attached to the lesser tuberosity. These tuberosities are in a fixed position with respect to the centre of rotation of the humeral head. The muscle forces of the rotator cuff act over an angle of about 270° (postero-, supero- and anterior quadrants) of the outer edge of the hemisphere of the humerus head as shown schematically in Fig. 3. In complex proximal humeral head fractures the broken bone fragments are retracted by the muscles but solid bone to tendon fixation still persists. Failure to restore the force couple leads to imperfect biomechanical function of the glenohumeral joint. The loss of the centring of the humeral head in the glenoid produces translation and/or rolling thereby altering the final outcome of the surgical treatment negatively.
2) When the length of the restored humerus is shortened, the deltoid muscle is constantly engaged in drawing the prosthetic head upwards. This explains why inferior subluxation is visible on early post-operative X-rays and not on later ones. This permanent contraction of the deltoid and shortening of the muscle fibres is sufficient to explain the reduction in active mobility which is often associated with chronic pain.
   Conversely, any elongation of the humerus causes excessive tension on the supaspinatus muscle and the structures of the subacromial arc, i.e. the anterior part of the acromion and the coracoacromial ligament. This predisposes a second rupture of the rotator cuff, once again with pain and often a reduction in active mobility.
   Restoration of the correct humeral length is thus essential for the optimum operation of the deltoid muscle and the greater tuberosity must be brought 5 to 10 mm below the prosthetic head. There should be no overlap or diastase of the tuberosities and the diaphysis below. All this is difficult to achieve in practice with known prostheses.
3) The restoration of the individually determined humeral retroversion has to be restored based on peroperative landmarks. This is however difficult with four-part fractures as the normal landmarks of the humeral anatomical neck and/or the biciptal groove have been lost. The success of the approximate choice of retroversion depends upon the skill and experience of the surgeon. The usual tendency has been for over-retroversion of the prosthesis which alters the centre of rotation and the balance of the rotator force couples resulting in poor active shoulder function. The generally recommended value of 35 to 40° of retroversion appears to be too high. Recent studies using digitised measurement of fresh human cadavers indicates that 20° of retroversion seems more reasonable to restore normal anatomy.
4) Sutured tuberosities may cause many peroperative and postoperative problems. An anatomical reduction of the tuberosities around the humeral head appears to be obligatory to restore a good balance of the rotator cuff force couples. A tendency to overtighten the sutures around the tuberosities which are most severely comminuted seems to exist.
5) Restoration of the medial offset depends upon the prosthesis itself which must match the anatomy as far as possible in terms of its design and must have sufficient choice of prosthetic head thickness to replace the fractured head. This implies a range of prosthetic heads from which the surgeon may choose the best fit, i.e. it implies the use of a modular prosthesis.
6) A restoration of medial and/or posterior offset with less than 5 mm displacement will not later the functional outcome of the treatment. A restoration of the valgus/varus orientation of the humeral head with less than 10° of displacement will also not alter the outcome. Therefore a standard 130° of valgus of the prosthetic head without adaptation of the posterior offset seems to be acceptable.
7) Patients with humeralplasties for fracture of the proximal humerus often show early displacement, osteolysis and pseudoarthrosis of the tuberosities. Early displacement of the tuberosities can be explained by the forces exerted by the tendon of the supraspinatus muscle which may reach 300 Newtons at 30 degrees abduction along the tendon.

Axillary lateral X-rays of the shoulder taken later reveal that there may be osteolysis of the tuberosities and in particular pseudoarthrosis between the tuberosities and the humeral diaphysis.

These problems can be resolved by not using cement in the metaphyseal tunnel and its replacement with cancellous bone graft taken from the humeral head. However, the more grafts are placed beneath the tuberosities, the more these tuberosities are pushed from the prosthetic head and its rotation centre, and the more the balance of the rotator cuff force couples may be disturbed even when flexible non-absorbable large diameter sutures through some holes on the prosthetic stem are used.

Summarising the above, conventional shoulder prostheses have significant shortcomings.

It is an object of the present invention to provide a prosthesis which overcomes the shortcomings of the known prostheses.

It is a further object of the present invention to provide a prosthesis whose successful use may be achieved by a normally skilled surgeon.

It is a further object of the present invention to provide a prosthesis which restores the rotator force couple correctly.

It is a further object of the present invention to provide a prosthesis with which the length of the broken proximal humerus can be measured preferably peroperatively in a simple and correct way.

It is a further object of the present invention to provide a prosthesis with which the particularity of the individual humeral retroversion can be restored based on peroperative landmarks.

It is a further object of the present invention to provide a prosthesis with which the tendency to overtighten the sutures around the tuberosities can be avoided thus also avoiding loss of the rotator cuff force couple.

It is a further object of the present invention to provide a prosthesis with which an adequate medial offset can be restored.

It is a further object of the present invention to provide a prosthesis with which a variability of the valgus/varus or posterior offset can be taken into account.

It is a further object of the present invention to provide a prosthesis with which possible osteolysis, malunion and/or pseudoarthrosis of the tuberosities can be avoided.

### SUMMARY OF THE INVENTION

The present invention provides a prosthesis as defined in claim 1.

The elements of the prosthesis may be made from any suitable material, e.g. a titanium-aluminium-vanadium alloy in accordance with ISO 5382/3, 1990 12/01.

The present invention differs from known prostheses for proximal humeral fracture in that the reconstruction of the proximal humerus starts at the glenoid. In accordance with the present invention the restoration of the caput humeri and its surrounding rotator cuff tendon in reconstructive surgery of the glenohumeral joint is made with respect to the glenoidal anatomy. The modular head of the prosthesis may be designed so that the surgeon fixes the broken tuberosities, to which the tendons are firmly attached, on the rim of a hemisphere or spherical cap, particularly a minor spherical cap, in an anatomical way in order to restore the rotator cuff couple correctly.

Further, the prosthesis may have a low bulk. This allows the use of bone grafts in the metaphyseal tunnel with considerably reduced risk of pushing the tuberosities off from the head and its rotation centre. In particular, the inside of the prosthetic head is preferably substantially hollow.

The dependent claims define further individual embodiments of the present invention. The present invention will now be described with reference to the following drawings.

### BRIEF DESCRIPTION OF THE DRAWINGS

Fig. 1 is a schematic representation of the typical bone locations of a fractured human shoulder.
Fig. 2 shows a cross-section of the gleno-humeral joint at the centre of rotation.
Fig. 3 shows a cross-section through the collum anatomicum of the proximal humerus.
Fig. 4 is a schematic representation of a prosthetic head in accordance with one embodiment of the present invention.
Fig. 5 is a schematic representation of a prosthetic shaft element in accordance with one embodiment of the present invention.
Fig. 6 is a cross-sectional schematic representation of a prosthetic head joined to a prosthetic shaft element in accordance with one embodiment of the present invention.
Figs. 7A and B are a schematic representations of an ancillary element for use with a shaft element of Fig. 5 according to one embodiment of the present invention.
Figs. 8, 9, 10A to C, 11 and 12 show schematic representations of surgical steps.
Fig. 13 is a schematic representation of a prosthetic head in accordance with another embodiment of the present invention.
Fig. 14 is a schematic representation of a prosthetic shaft element in accordance with another embodiment of the present invention.
Fig. 15 is a schematic representation of an ancillary element for use with a shaft element of Fig. 14.

### DESCRIPTION OF THE ILLUSTRATIVE EMBODIMENTS

The present invention will be described with reference to certain embodiments and particular drawings but the invention is not limited thereto but only by the claims. In particular the present invention will mainly be described with reference to a prosthesis for restoration after a humeral fracture but it may also find application as a prosthesis in other types of reconstructive surgery for other joints. For example, the prosthesis according to the present invention may be used in the restoration of the glenohumeral joint for humans suffering from degenerative or inflammatory arthritis, including the use with a further glenoidal prosthetic element for restoration of the glenoid, which further prosthetic element may include either a hemispherical cavity or a convex hemisphere.

Figs. 4 to 7 are schematic representations of part of a kit of parts used for assembly of a modular anatomical prosthesis in accordance with a first embodiment of the present invention. Fig. 4 is a schematic representation of a first prosthetic element 10 which is used for the restoration of the caput humeri. Preferably element 10 is a single piece as shown but this element 10 may also be made up of two or more pieces which may be joined together. The element 10 has a convex, substantially hemispherical or spherical cap form 11 which may be solid but its inner region 11A is preferably substantially hollow in accordance with the present invention. A spherical cap is the region of a sphere which lies above a given plane. If the plane passes through the centre of the sphere a hemisphere is generated. In accordance with all the embodiments of the present invention it is preferred if element 10 has a hemispherical or minor spherical cap form. A minor spherical cap is used in the present invention to mean a spherical cap whose depth is less than the radius, i.e. it is smaller than a hemisphere. Conversely, a major spherical cap is larger than a hemisphere. In accordance with the present invention a spherical cap refers to a convex or concave (= cavity) structure unless otherwise shown or described.

The hemispherical or spherical cap form 11 is adapted to co-operate with the glenoid of the shoulder or with a glenoidal prosthetic element attached to the glenoid and used as a restoration thereof as is known from reconstructive surgery of the glenohumeral joint for degenerative or inflammatory arthritis.

Around the rim of the hemisphere or spherical cap 11, suture attachment means 14 are provided. These may be a plurality of holes 14 as shown or hooks or slits or any other suitable structure for threading of sutures spaced around the rim. The holes 14 are preferably spaced around an angle of 270° of the rim of hemisphere or spherical cap 11. Preferably 7 holes are provided. The rim of the hemisphere or spherical cap may be flat or may undulate or be sculptured. Further, the holes 14 are located on the rim. It is preferable in accordance with the present invention if the rim of the hemisphere or spherical cap 11 lies substantially in one plane and it is preferred if this plane is substantially parallel to or coincides with the plane of the anatomical neck of the humerus and not the surgical neck. It is preferred in accordance with the present invention if the suture attachment holes 14 lie as close as possible to the rim.

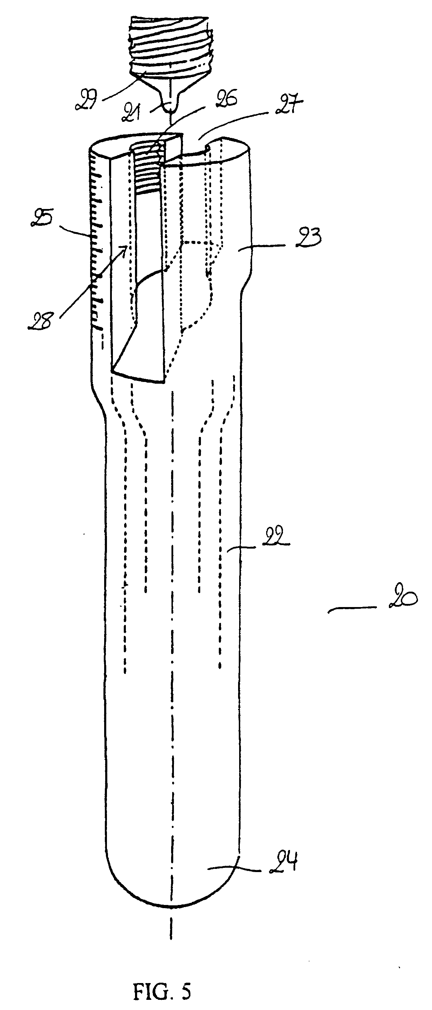

Within the hemisphere or spherical cap 11 a means is provided for connection to a shaft element 20 of the prosthesis. The connection means may be a rod-like structure 12 which is firmly attached to the hemisphere or spherical cap and extends therefrom in a direction remote from the outer surface of the hemisphere or spherical cap 11. The rod 12 preferably extends parallel to the axis of the hemisphere or spherical cap which is perpendicular to the plane through the rim of the sphere which defines the hemisphere or spherical cap. The rod 12 serves to attach the first element 10 to the shaft element 20 shown schematically in Fig. 5. Rod 12 may include a depression 13 which provides a method of fixing the first element 10 to the shaft element 20 in a non-rotatable manner as will described later. Rod 12 may be circular in cross-section but other cross-sections are possible, e.g. square. The radius of the hemispherical or spherical cap form 11 may be chosen by the surgeon from a series of prosthetic elements 10 of different standard sizes. For instance, a kit may include a plurality of first elements 10 with diameters "c" and depth from crown to rim of (c/depth): 39/14, 41/15, 43/16, 46/17, 48/18, 50/16 and 50/19 mm respectively. The distance "b" from the crown to the depression 13 of these elements 10 may be 46, 48, 50, 53, 55, 57 and 57 mm respectively.

Fig. 5 is a schematic representation of a shaft element 20 in accordance with the first embodiment. Again it is shown as a single piece but is may also be made of several joinable pieces. Shaft element 20 includes a substantially cylindrical element 22 having an upper or proximal end 23 and a lower or distal end 24. Element 20 may be provided in several standard sizes, e.g. diameters of 6, 9 and 12 mm. The distal end 24 is shaped in such a manner that it may be inserted into the inner channel of the humerus bone. The proximal end 23 includes an inclined slit 28 for receiving rod-like stem 12 of the first element 10. The inclination of slit 28 is preferably about 40° to the horizontal. Located in the proximal end 23 of the element 20 is a longitudinal bore 27 having a screw-thread 26 over at least part of its length. Screw-thread 26 is provided for receipt of a set screw 29 for firmly securing rod 12 within slit 28. Set screw 29 may include a shaped end piece 21 for location in the depression 13 of rod 12. The proximal end 23 may be provided with calibration markings 25 starting from the end thereof.

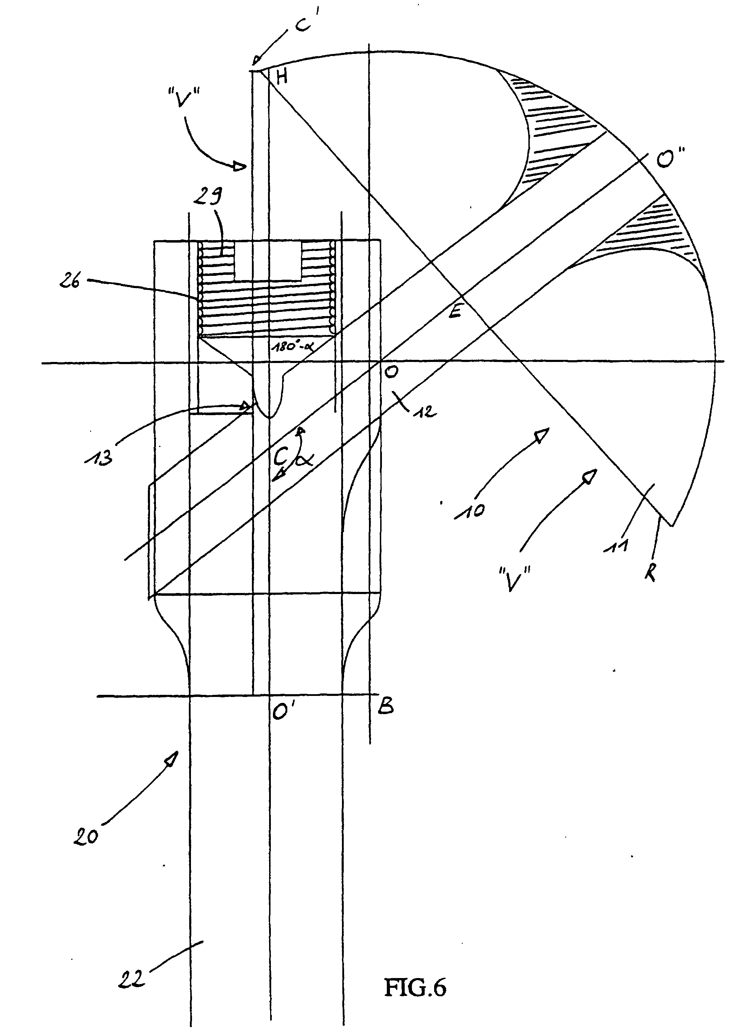

The assembly of the first and shaft elements 10 and 20 is shown schematically in cross-section in Fig. 6. In accordance with the present invention the shaft element 20 and the cap 11 co-operate in such a way when joined together that a circumferential free volume "V" is provided between the rim R of the cap 11 and the outer diameter of the shaft element 20. The volume "V" is used in accordance with the present invention for the accommodation of the tuberosities or parts thereof as well as for the introduction of bone graft. Accordingly, the cap 11 is terminated at the rim R on which the suture attachment means 14 are provided and the rim forms a shoulder or step between the rim and the shaft element 20. Preferably the cap 11 is hollow to allow introduction of bone graft.

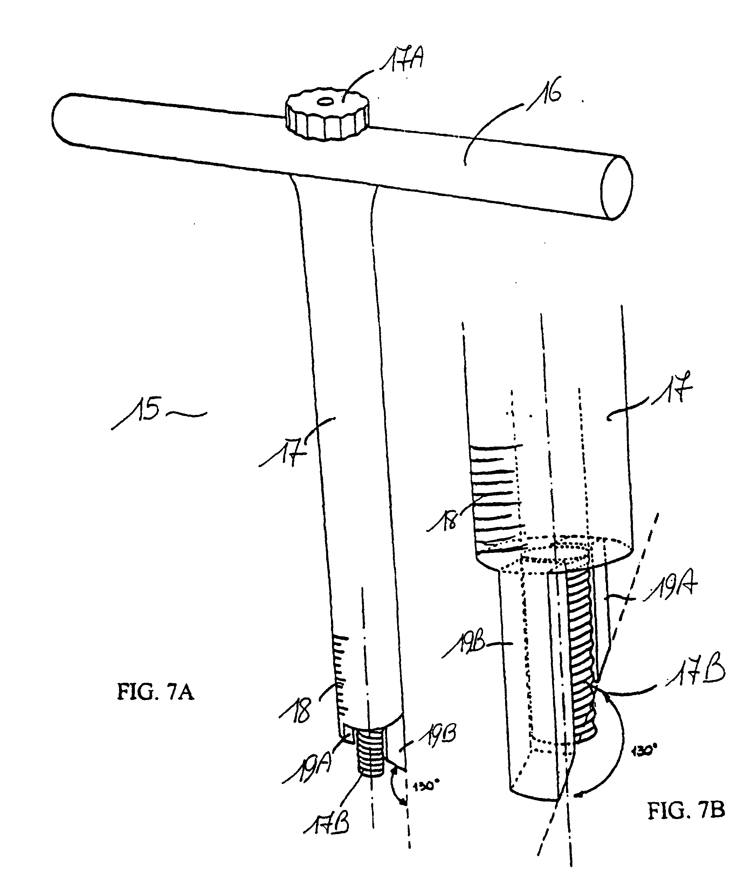

Fig. 7A is a schematic representation of an ancillary element 15 according to the first embodiment. Fig. 7B is a detailed view of the lower end of element 15. The ancillary element 15 is used to assist in placing the shaft element 20 in the channel of the humerus at the correct height to restore the correct humeral length. Ancillary element 15 may include a cross-member 16 which is preferably shaped so that the surgeons hands may grip it easily and direct both downwards and rotational forces thereto accurately and controllably. Cross-member 16 is preferably attached to a vertical elongate member 17. Elongate member 17 is preferably cylindrical having the same diameter as the proximal end 23 of shaft element 20. The lower end of member 17 may be provided with calibration markings 18 which align with the calibration markings 25 of the shaft element 20 when the two are attached together. The lower end of member 17 also has two diametrically opposed guide members 19A and 19B which are of such a width that they slide into the slit 28 of the shaft element 20 and are of different length so that the fit exactly into the inclination of slit 28. When the ancillary element 15 is located in the shaft element 20, the cross-member 16 is perpendicular to the length of the slit 28. Cross-member 16 is preferably oriented perpendicular to line passing through the two guide members 19A and 19B. Down the hollow centre of ancillary element 15 runs a long bolt having a knurled head 17A at its upper end and a screw thread 17B at its lower one. The screw-thread 17B of the bolt is engageable with the screw thread 26 of shaft element 20.

Ancillary element 15 may be located in the slit 28 of the shaft element 20 making sure that the guide members 19A and 19B locate correctly in the slit 28. The knurled head 17A of the bolt is then turned to introduce its lower screw-thread 17B into the screw thread 26 of the shaft element 20 thus fixing the two together in a predetermined and repeatable geometrical position with respect to each other.

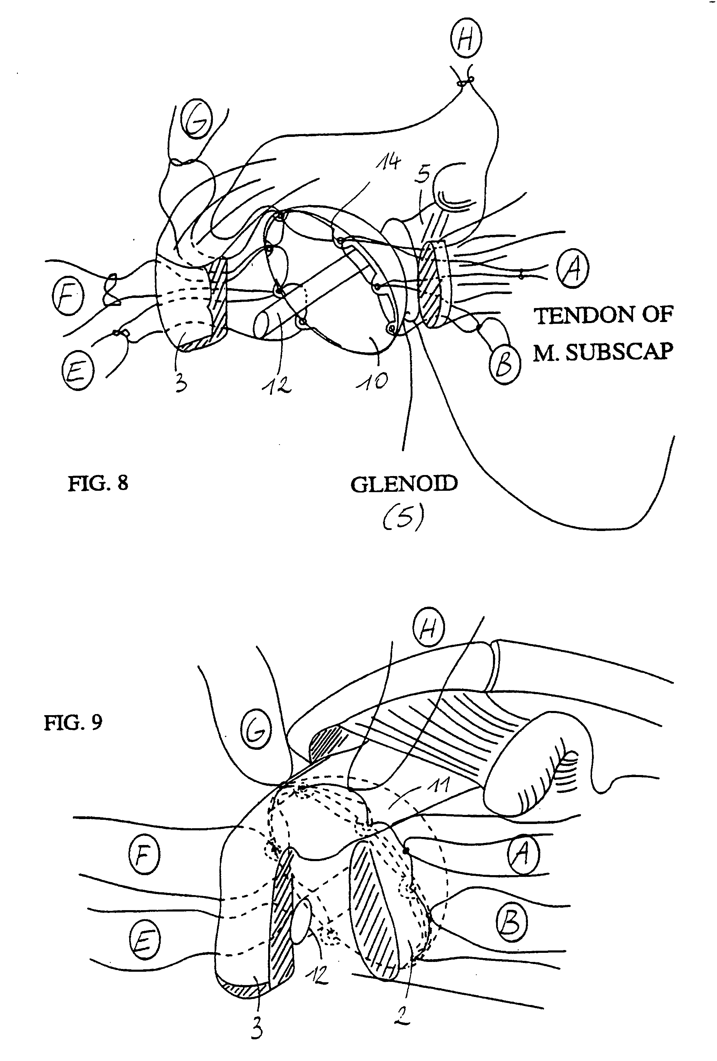

An embodiment of the device in accordance with the present invention will now be described with reference to Figs. 8 to 12. In a first step as shown schematically in Fig. 8 the tendons are fixed temporarily to the first element 10. Flexible non-absorbing sutures A, B, E, F, G and H are threaded through the holes 14 of element 10 so as to loosely hold this element 10 approximately with respect to the glenoid 5. The outer hemispherical or spherical cap form 11 is directed towards the glenoid 5. The sutures attach the following tendons: A, B: subscapularis; E: teres minor and infraspinam; F, G: supraspinatus; and H: a suture across the rotator cuff interval. As shown schematically in Fig. 9, in a second step the sutures A, B, G and H are knotted tightly. This brings element 10 against the glenoid and draws the larger and smaller tuberosities 3, 2 to the hemispherical or spherical cap element 10. In accordance with the present invention bone parts such as the smaller and larger tuberosities are not removed from their tendons but rather these bone elements are incorporated into the restored shoulder joint. To achieve this the space V is provided for accommodation of these bone portions as shown in Fig. 9.

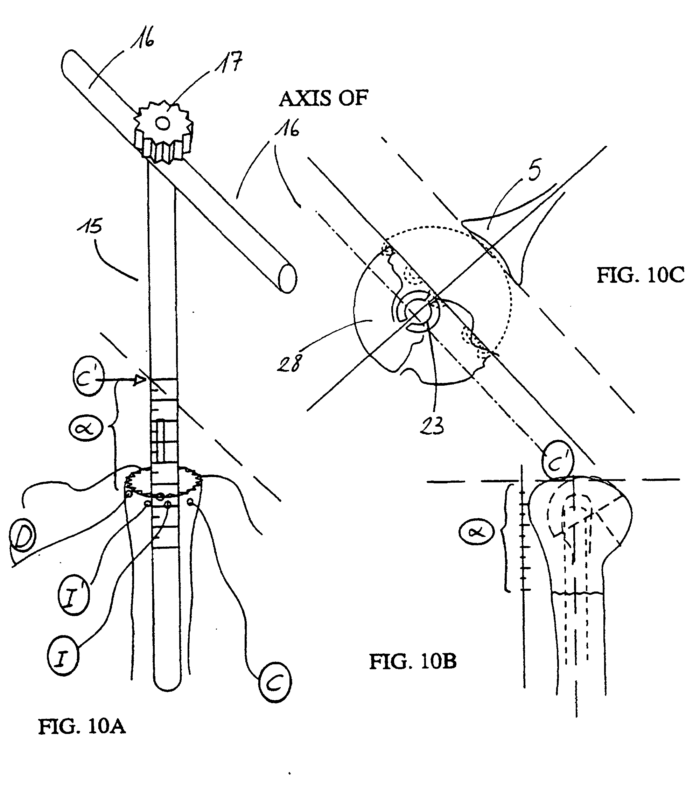

In a third step as shown schematically in Fig. 10A. The humeral channel is reamed out in its proximal side. By using reamers of progressively larger size, e.g. 6, 9, 12 mm, the final size of the prosthetic stem 20 is determined. The selected shaft element 20 is attached to the ancillary element 15. The dimension "a" in Fig. 10B from the top of the humerus (sometimes known as the "hinge point") to the fracture line between the caput humeri and the diaphysis is measured (= the depth of the greater tuberosity) and based on this measurement and the known geometry of the completed prosthesis, the calibration mark "C" of the calibrations 25 or 18 is calculated which comes level with the top of the fractured diaphysis 1 when the shaft element 20 is at the correct depth in the channel of the diaphysis 1. It is preferred in accordance with the present invention if the upper zero mark of the calibration marking 18 (or 25 if there is no calibration 18 on the ancillary element 15) is located at the point which allows the correct position of the prosthetic shaft 20 to be determined merely by translating downwards by the distance "a". Keeping the arm in a neutral position, the ancillary element 15 with attached shaft element 20 is then inserted into the channel of the diaphysis to the correct depth while keeping the cross-member 16 parallel with plane of the glenoid 5 as shown schematically in Fig. 10C. This effectively makes the centre axis of the glenoid parallel with the axis of the prosthetic head 10 allowing for adaptation of the individual retroversion. Then, the knurled head of the bolt 17 is released and the proximal end 23 of shaft element 20 is offered up to the rod 12 of element 10. Holes are then bored in the end of the diaphysis to receive the sutures C, D, and I which are left loose. The shaft element 10 may now be cemented into the diaphysis.

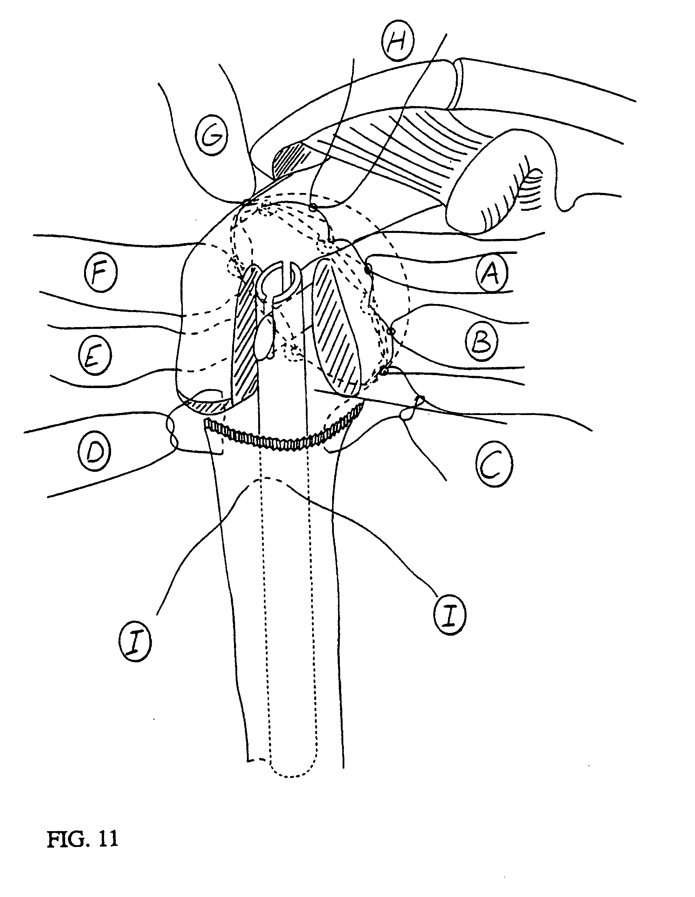

As shown schematically in Fig. 11, rod 12 is located in slot 28 of shaft element 20. The set screw 29 is screwed into screw-thread 26 and tightened into the depression 13 of rod 12 (set-screw is omitted from the drawing for clarity purposes). The element 10 is now locked to the shaft element 20 in the correct anatomical position. Flexible non-absorbable sutures C and D are threaded between the humerus diaphysis 1 and the tuberculum minus via a hole 14 of element 10 and between the humerus diaphysis 1 and the tuberculum magnus but not tightened.

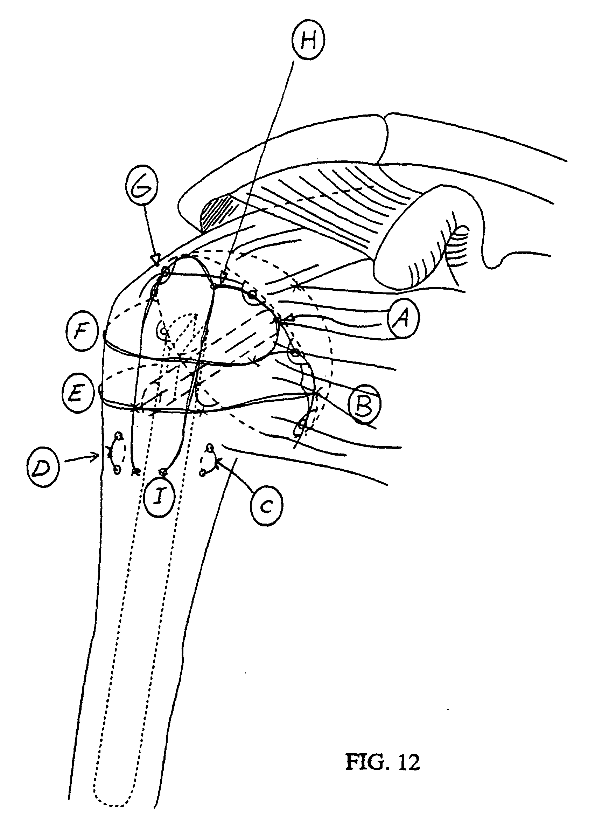

The final step is shown schematically in Fig. 12. Bone graft is introduced into the hollow element 10 and packed around it. The remaining sutures are tightened as well as the application of restraining sutures B-E, F-E, I-G and I-H to provide additional lateral and vertical stability to the restoration and a good compression of the bone grafting by this tension band wiring of the tuberosities.

Alternatively, the sutures A, B, E, F, G, and H are left loose until after the shaft element 10 has been firmly fixed. Then the sutures C and D are completed and tied firmly. Then the bone grafting is done and finally a multiple tension band wiring is performed by tying suture A to F (or E), B to E (or F) and G and H to I.

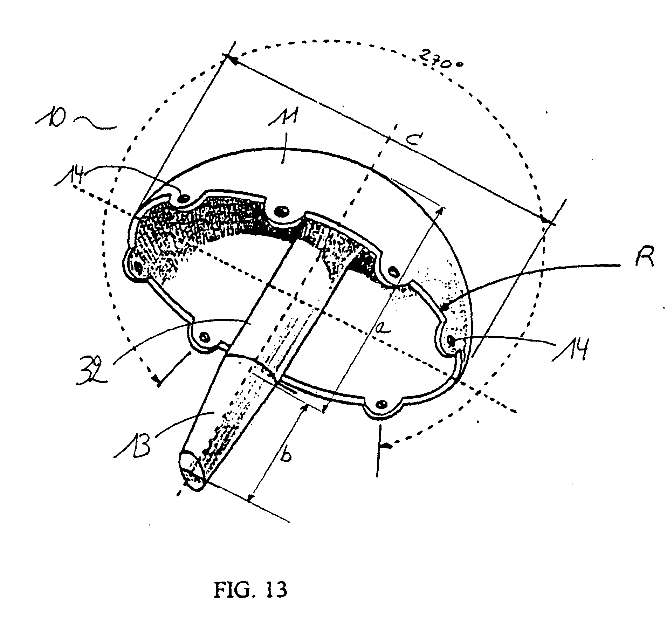

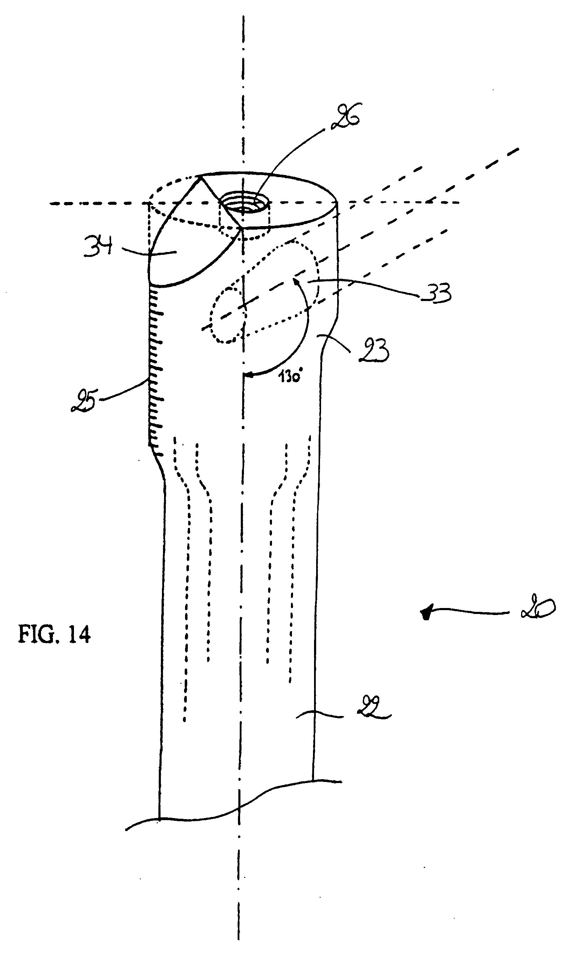

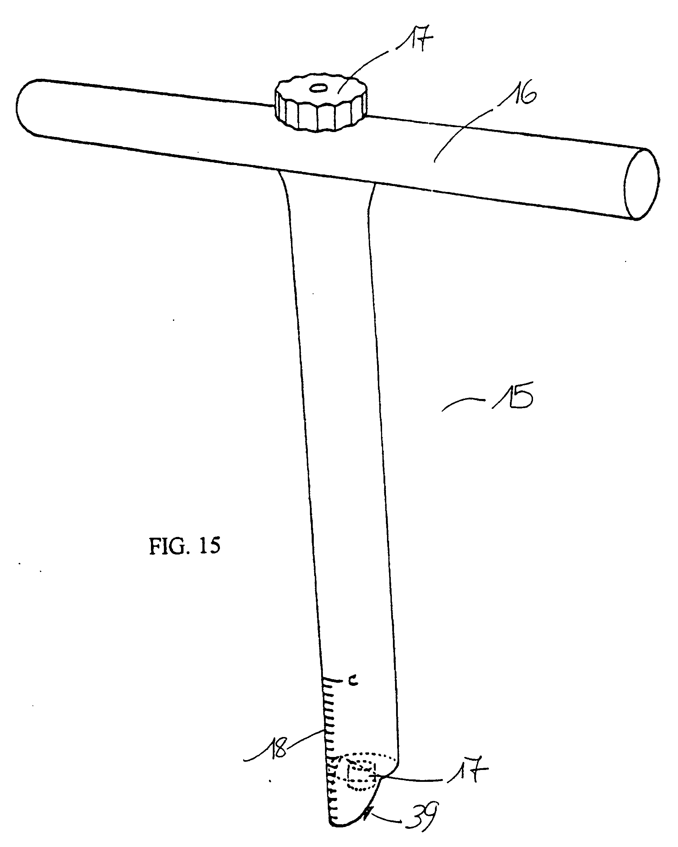

A further embodiment of a kit for a prosthesis in accordance with the present invention will be described with reference to Figs. 13 - 15. The only major difference between this embodiment and the embodiment described with reference to Figs. 4 to 7 is the method of securing the rod 12 of the first element 10 to the shaft element 20. As shown schematically in Fig. 13 the first element 10 has a hemispherical or spherical cap member 11 and suture attachment points 14 arranged around the circumference of the element 10. The hemisphere or spherical cap 11 is preferably substantially hollow. A cylindrical rod 32 is attached to and extends from the inner side of the hemispherical or spherical cap form 11. At the end of the rod 32 there is a device for fixing the first element 10 to the shaft element 20, in this embodiment a Morse tapered section 13 which may be received into a matching recess 33 in the shaft element 20 as shown schematically in Fig. 14. To prevent rotation, the Morse taper 13 may be keyed into the recess 33. The shaft element 20 has a screw-threaded bore 26 in its upper end and a flat 34 placed at a suitable angle which will be used to align the ancillary element 15 shown schematically in Fig. 15. The ancillary element 15 in accordance with this embodiment has a cross-member 16 and bolt which passes through the body 17 of ancillary element 15 and has an upper knurled head 17A and a threaded lower end 17B. The lower end of body 17 has a protruding flat 39 which mates and aligns with the flat 34 of the shaft element 20 when they are fixed together. Body 17 includes calibration markings 18 which align with calibration markings 25 on the shaft element 20 when they are fixed together.

Except for these modifications, the prosthesis and the kit are used as described above for the previous embodiment. In particular, this embodiment also provides a volume or space V between the rim R of the cap 11 and the shaft element 20 for the accommodation of bony material. Further, in both embodiments the plane in which the rim R of the cap 11 lies, is parallel or coincides with the anatomical neck of the humerus and not with the surgical neck. Hence, the connecting means between the cap 11 and the shaft element 20 holds central axis of the cap 11 at the correct anatomical angle and the cap and shaft are joined together by movement along the central axis of the cap 11. The cap 11 of both embodiments may be described as basically mushroom shaped. The rim R of the cap 11 forms with the outer surface of the shaft element 20 a neck, i.e. a reduction in diameter, the neck being formed by the rod 12, 32 and the end of the shaft element 20. It is this neck which provides the volume V. It is preferred in accordance with any embodiment of the present invention if the suture attachment means 14 are located at or adjacent to the diameter of the sphere of which the cap 11 forms a part. This means that it is preferred if the suture attachment means 14 and/or the rim R are not placed more distally from the top of the cap 11 than the diameter of the sphere of which the cap 11 forms a part.

The prosthesis in accordance with the present invention restores the force couple of the rotator cuff in an anatomical way. The tendons of the rotator cuff are anchored anatomically at their bony transitions over 270°, i.e. over the posterior, superior and anterior quadrants, of the rim of the prosthetic head which is designed as an anatomical, adaptable and modular prosthesis (typically 7 sizes). The humeral stem of the prosthesis is adapted to avoid humeral shortening. The total length of the broken tuberculum majus and its translation to the measurable position of the humeral stem is measured peroperatively. The ancillary placement tool and/or the humeral stem of the prosthesis is calibrated with markings which allow exact placing of the depth of the humeral stem in the channel of the diaphysis. This avoids the need for peroperatively taking X-rays of the contralateral shoulder which may be painful for the patient. Parallelism of the axis of the humeral anatomical neck and the axis of the glenoid when the forearm is in a position of neutral and external rotation provides an easy to perform and controllable positioning of the retroversion of the humeral stem. The possibility of overtightening of the sutures is reduced or eliminated because of their solid fixation to the rim of the prosthetic head. The modular approach of the prosthesis in accordance with the present invention allows for a an average medial offset for each size. A fixed 130° angulation of the humeral head to the stem has been chosen to avoid valgus or varus deformity. The attachment of the rotator cuff tendons at their bony transitions to the rim of the prosthetic head also allows autologous bone grafting from the humeral head without pushing away the tuberosities. Even more bone grafting can be used because of the cavity in the humeral head prostheses as well as the minimal bulkiness of the prosthetic device. Moreover, a good compression of the grafting by the tension band wiring of the tuberosities can be achieved resulting in less pseudoarthrosis and osteolysis of the tuberosities.

Although the invention has been described in detail with reference to preferred embodiments and specific constructions and methods, the invention is not limited to these and the skilled person will appreciate that there are modifications and variations within the scope of the present invention as defined in the attached claims. For instance, the first element 10 may include an internally threaded boss on its inner surface and the shaft element 20 may be joined to the first element 10 by means of a bolt passing through an inclined threaded bore in the shaft element 20. Further, the first element 10 may be made solid and the suture attachment means 14 may be provided by holes close to the rim of the first element 10. Alternatively, the shaft element 20 may include a plate angled at 50° to the horizontal and this may be fixable to the underside of the first element 10 as is generally known from US 5,314,479 or EP-A-0 549 480.

In addition the prosthesis in accordance with the present invention may be joined to the humerus by other means than insertion of a shaft element into the diaphysis. For instance, the shaft element 20 may be adapted so that it may be joined to the humerus by any suitable means, e.g. screwed or pinned, or cemented to the humerus, and the length and shape of the shaft element 20 may be changed as required provided the volume V is provided for accommodation of the bony parts of the fractured shoulder joint. In addition the cap 11 may be joined to the shaft element 20 in such a way that the angle between the central axis of the cap 11 and the shaft element 20 is variable as is known in principle (without the inventive features of the present invention) from US 5702457 by using a sphere journelled in a shaft element.

## Claims

1. A prosthesis for a shoulder joint for use between an anatomical cavity having a substantially hemispherical or spherical cap form and an upper end of an elongate bone having an inner channel,
wherein the prosthesis comprises :
- a first part having a substantially hemispherical or spherical cap form (11) adapted to cooperate with the anatomical cavity having the hemispherical or spherical cap form, and
- a second part in the form of a stem (20) for fixing with the elongate bone,
- an annular volume (V) is provided between the first and second parts for the receipt of bone grafts ;
**characterised in that** :
- said first part includes a plurality of attachment devices (14) for attaching sutures;
- the suture attachment devices (14) are located as close as possible to the circular rim (R) of the hemispherical (11) or spherical cap form of said first part;
- said first and second parts being adapted so that said first part is fixable with respect to the anatomical cavity having the hemispherical or spherical cap form independently of the fixation of said second part with the elongate bone.

2. A prosthesis according to claim 1, wherein said first part is a substantially hollow hemispherical or spherical cap.

3. A prosthesis according to any of claims 1 to 2, wherein said first part includes a stem insertable in said second part.

4. A prosthesis according to any of claims 1 to 3, wherein the hemispherical or spherical cap structure is a further prosthesis element attachable to a second bone.

5. A prosthesis according to claim 4, wherein the further prosthesis element has a convex outer form of a hemispherical or spherical cap.

6. A prosthesis according to any of claims 1 to 5, wherein said first part is non-rotatably fixable to said second part.

## Patentansprüche

1. Prothese für ein Schultergelenk zum Einsatz zwischen einem anatomischen Hohlraum mit einer im wesentlichen hemisphärischen oder sphärischen Kappenform und einem oberen Ende eines ausgestreckten Knochens mit einem Innenkartal,
wobei die Prothese folgendes besitzt :
- einen ersten Teil mit im wesentlichen hemisphärischer oder sphärischer Kappenform (11), die mit dem anatomischen Hohlraum, der die hemisphärische oder sphärische Kappenform besitzt, kooperieren kann, und
- einen zweiten Teil in Form eines Schaftes (20) zur Befestigung an den ausgestreckten Knochen,
- ein ringförmiges Volumen (V) ist zwischen dem ersten und dem zweiten Teil für die Aufnahme von Knochentransplantaten vorgesehen ;
**dadurch gekennzeichnet, dass** :
- der erste Teil eine Vielzahl von Befestigungsvorrichtungen (14) zur Befestigung von Nähten besitzt;
- sich die Befestigungsvorrichtungen für die Nähte (14) so nahe wie möglich an dem kreisförmigen Rand (R) der hemisphärischen (11) oder sphärischen Kappenform des ersten Teils befinden;
- der erste Teil und der zweite Teil so ausgebildet sind, dass der erste Teil, unabhängig von der Befestigung des zweiten Teils an dem ausgestreckten Knochen, an dem anatomischen Hohlraum befestigt werden kann, der die hemisphärische oder sphärische Kappenform besitzt.

2. Prothese nach Anspruch 1, wobei es sich bei dem ersten Tel um eine im wesentlichen hohle hemisphärische oder sphärische Kappe handelt.

3. Prothese nach einem der Ansprüche 1 bis 2, wobei der erste Teil auch einen Schaft besitzt, der in den zweiten Teil eingeschoben werden kann.

4. Prothese nach einem der Ansprüche 1 bis 3, wobei es sich bei der hemisphärischen oder sphärischen Kappenstruktur um ein weiteres Protheseelement handelt, das an einem zweiten Knochen befestigt werden kann.

5. Prothese nach Anspruch 4, wobei das weitere Prothesenelement die konvexe äußere Form einer hemisphärischen oder sphärischen Kappe besitzt.

6. Prothese nach einem der Ansprüche 1 bis 5, wobei der erste Teil nicht drehbar an dem zweiten Teil befestigt werden kann.

## Revendications

1. Prothèse destinée à une articulation d'épaule pour une utilisation entre une cavité anatomique ayant une forme de chapeau sensiblement hémisphérique ou sphérique et une extrémité supérieure d'un os allongé ayant un canal intérieur,
la prothèse comportant :
- une première partie ayant une forme de chapeau sensiblement hémisphérique ou sphérique (11) adaptée pour coopérer avec la cavité anatomique ayant la forme de chapeau hémisphérique ou sphérique, et
- une seconde partie ayant la forme d'une tige (20) pour fixation avec l'os allongé,
- un volume intérieur (V) étant fourni entre les première et seconde parties pour la réception de greffons osseux,
**caractérisée en ce que** :
- ladite première partie comporte une pluralité de dispositifs de fixation pour fixation de fils de suture,
- les dispositifs de fixation de fils de suture (14) sont positionnés aussi proche que possible du pourtour circulaire (R) de la forme de chapeau hémisphérique (11) ou sphérique de ladite première partie,
- lesdites première et seconde parties sont adaptées de sorte que ladite première partie peut être fixée par rapport à la cavité anatomique ayant la forme de chapeau hémisphérique ou sphérique indépendamment de la fixation de ladite seconde partie avec l'os allongé.

2. Prothèse selon la revendication 1, dans laquelle ladite première partie est un chapeau hémisphérique ou sphérique sensiblement creux.

3. Prothèse selon la revendication 1 ou 2, dans laquelle ladite première partie comporte une tige pouvant être insérée dans ladite seconde partie.

4. Prothèse selon l'une quelconque des revendications 1 à 3, dans laquelle la structure de chapeau hémisphérique ou sphérique est un élément de prothèse supplémentaire pouvant être fixé sur un second os.

5. Prothèse selon la revendication 4, dans laquelle l'élément de prothèse supplémentaire a la forme extérieure convexe d'un chapeau hémisphérique ou sphérique.

6. Prothèse selon l'une quelconque des revendications 1 à 5, dans laquelle ladite première partie est fixée sur ladite seconde partie de manière non-rotative.
